# EUROPÄISCHE PATENTSCHRIFT

(11) **EP 2 691 030 B1**
(45) Veröffentlichungstag und Bekanntmachung des Hinweises auf die Patenterteilung: **07.07.2021**
(21) Anmeldenummer: 12707777.4
(22) Anmeldetag: 09.03.2012
(51) Int. Cl.: A61B 17/122, A61B 17/128

(54) **CHIRURGISCHER CLIP-APPLIKATOR**
SURGICAL CLIP APPLICATOR
INSTRUMENT DE POSE D'AGRAFES CHIRURGICALES

(30) Priorität: 31.03.2011 DE 102011001705
(43) Veröffentlichungstag der Anmeldung: 05.02.2014
(73) Patentinhaber: Aesculap AG, 78532 Tuttlingen (DE)
(72) Erfinder: SCHULZ, Peter, 79843 Löffingen (DE); MORALES, Pedro, 78532 Tuttlingen-Nendingen (DE); WEISSHAUPT, Dieter, 78194 Immendingen (DE)
(74) Vertreter: Hoeger, Stellrecht & Partner Patentanwälte mbB
(86) Internationale Anmeldenummer: PCT/EP2012/054107
(87) Internationale Veröffentlichungsnummer: WO 2012/130590

(56) Entgegenhaltungen:
- DE-U1-202006 000 329
- DE-U1-202011 000 754
- DE-U1-202011 000 755
- GB-A- 2 073 022
- US-A- 3 631 707
- US-A- 3 777 538
- US-A- 5 609 599
- US-B1- 6 258 105

## Beschreibung

Die Erfindung betrifft einen chirurgischen Clip-Applikator mit einem Doppelsteg- oder Mehrfachclip mit einer U- oder V-förmigen Clipstruktur jeweils mit jeweils zwei über einen Verbindungsbereich miteinander verbundenen Schenkeln. Der chirurgische Clip-Applikator umfasst einen Griff, einen sich an den Griff anschließenden Schaft und ein Anlegewerkzeug für die Clips am freien Ende des Schafts, welches mit dem Griff betätigbar ist und zwei beim Betätigen aus einer offenen Ruhestellung in eine Schließstellung überführbare Werkzeugbacken aufweist. Die Werkzeugbacken weisen jeweils einen Aufnahmebereich für den zu applizierenden Clip auf und die Clips werden häufig in einem Clip-Magazin vorgehalten, welches beispielsweise am Schaft des Clip-Applikators gehalten wird.

Ein solcher chirurgischer Clip-Applikator für einfache U-förmige Clips ist z.B. aus der DE 19603889 A1 bekannt.

Neben einfachen U- oder V-förmigen Clips sind des weiteren auch sogenannte Doppelsteg- oder Mehrfachclips bekannt bei denen zwei oder auch drei Clips parallel nebeneinander in einem Arbeitsschritt zur Anlage an einer Gewebestruktur, beispielsweise an einem Blutgefäß, gebracht werden.

Bei den bekannten Clip-Applikatoren besteht das Problem, insbesondere bei der Verwendung von Doppelsteg- oder Mehrfachclips, dass die Kraft zum Schließen der Clips, die allein aufgrund des Clipmaterials aufgewendet werden muss, um die Clips sicher zu schließen bzw. sicher in Anlage an die Gewebestruktur zu bringen, erheblich ist und von dem operierenden Chirurgen einen entsprechenden Kraftaufwand verlangt.

Aus dem US Patent US 3,777,538 A ist ein chirurgisches Instrument bekannt, das zur Anbringung von Clips an schwer zugänglichen Stellen konzipiert ist.

Das Instrument beinhaltet einen lang gestreckten Schaft, an dem am proximalen Ende ein Griff montiert ist, während am distalen Ende Werkzeugbacken für die Applikation der Clips vorgesehen sind.

Aus dem US Patent US 5,609,599 A ist ein Clip-Applikator bekannt, der Werkzeugbacken mit einer Ausnehmung aufweist, in welchen ein zu applizierender Clip aufgenommen werden kann. Der zu applizierende Clip weist eine geschlossene O-Form auf und wird bei der Applikation hälftig zusammengebogen.

Aufgabe der vorliegenden Erfindung ist es, einen chirurgischen Clip-Applikator der eingangs beschriebenen Art vorzuschlagen, der das Anlegen und sichere Schließen der Clips mit einem verminderten Kraftaufwand erlaubt.

Diese Aufgabe wird durch einen chirurgischen Clip-Applikator mit den Merkmalen des Anspruchs 1 gelöst.

Aufgrund der Ausnehmung, die in den Anlageflächen der Werkzeugbacken vorgesehen ist, um den Verbindungsbereich des Clips aufzunehmen, kann der Clip bzw. dessen Schenkel an der Gewebestruktur, beispielsweise dem Blutgefäß, sicher in Anlage gebracht werden, ohne dass der Verbindungsbereich ebenfalls vollständig verpresst werden muss. Es verbleibt bei dem mit dem erfindungsgemäßen chirurgischen Clip-Applikator applizierten Clip im Verbindungsbereich ein kleiner offener Bereich, der typischerweise entweder von Gewebestruktur, beispielsweise der Wandung eines Blutgefäßes, gefüllt ist oder auch leer bleiben kann und den sicheren Verschluss eines Blutgefäßes nicht beeinträchtigt.

Der Clip wird damit im Verbindungsbereich in kleinerem Umfang verformt, so dass der Kraftaufwand beim Schließen des Clips vermindert ist, während die Schenkel soweit geschlossen werden, dass das geforderte Spaltmaß, bespielsweise ca. 0,25 mm oder weniger, insbesondere ca. 0,07 bis ca. 0,15 mm, eingehalten wird.

Diese Einsparung an Kräften bei der Verformung des Clips zum Schließen desselben ist in doppeltem oder mehrfachem Umfang gegeben, wenn Doppelsteg- bzw. Mehrfach-Clips zum Einsatz kommen.

Das Arbeiten mit dem erfindungsgemäßen chirurgischen Clip-Applikator ist dadurch wesentlich erleichtert, so dass auch umfangreichere Arbeiten wesentlich ermüdungsfreier durchgeführt werden können, während das geforderte Spaltmaß der geschlossenen Clips sicher erreicht werden kann.

Die Anlageflächen der erfindungsgemäßen chirurgischen Clip-Applikatoren können unterschiedlich ausgestaltet sein, wobei bei einer Ausführungsform sich die Anlageflächen von den freien Enden der Schenkel bis zu der Ausnehmung, die den Verbindungsbereich der Schenkel des Clips aufnimmt, gleichmäßig planar verlaufen. Hier entsteht eine Anlagefläche, die die Schenkel der Clips von ihrem freien Ende bis zum Verbindungsbereich gleichmäßig in Anlage an die Gewebestruktur bringt und so für eine möglichst plane Ausrichtung der beiden Schenkel im applizierten Zustand sorgt.

Bei einer alternativen Ausführungsform kann vorgesehen sein, dass bei dem erfindungsgemäßen Clip-Applikator die Anlagefläche im Bereich der freien Enden der Schenkel einen Rücksprung aufweist, so dass die Schenkel des Clips im mittleren Bereich, d.h. im Bereich zwischen den freien Enden und dem Verbindungsbereich sicher gefasst und zur Anlage an der Gewebestruktur, an der der Clip appliziert werden soll, gebracht werden.

Dies ist möglich, da aufgrund der V- oder U-förmigen Clipstruktur über die Anlageflächen ein Verschwenken der Schenkel des Clips um eine von dem Verbindungsbereich definierte Achse unter plastischer Verformung des Clip-Materials im Verbindungsbereich geschieht. Aufgrund dieser Verformung reicht eine Krafteinleitung im mittleren Bereich der Schenkel aus, um für eine sichere Anlage der gesamten Schenkel an der Gewebestruktur, insbesondere an einem Blutgefäß, zu sorgen.

Vorzugsweise sind dabei die Anlageflächen eines erfindungsgemäßen Clip-Applikators so bemessen, dass die Schenkel über ca. ein Drittel ihrer Länge oder mehr, insbesondere die Hälfte der Länge oder mehr, in der Schließstellung der Werkzeugbacken an diesen anliegen.

Bei einer weiteren bevorzugten Ausführungsform sind die Anlageflächen so bemessen, dass die Schenkel über ca. zwei Drittel ihrer Länge oder mehr in der Schließstellung der Werkzeugbacken an den Anlageflächen anliegen.

Die von den Aufnahmebereichen der Werkzeugbacken rückspringenden Ausnehmungen können unterschiedlich ausgebildet sein. Der Querschnitt, senkrecht zu der Längsrichtung der Schenkel der Clips gesehen, kann halbrund oder auch polygonal ausgestaltet sein, wobei der dabei erzielte Rücksprung gegenüber den Anlageflächen vorzugsweise jeweils so ist, dass in der Schließstellung der Werkzeugbacken keine Kraft auf den Verbindungsbereich des Clips ausgeübt wird.

Der Verbindungsbereich ist dabei vorzugsweise als eine Strecke vom Scheitelpunkt des Clips in Richtung zum freien Ende eines Schenkels des Clip-Materials definiert, die ungefähr dem Vierfachen oder weniger, insbesondere etwa dem Dreifachen des Durchmessers oder der Dicke eines Clip-Schenkels entspricht.

Unter der Dicke eines Clip-Schenkels wird vorliegend dessen Ausdehnung in Schließrichtung des Clips verstanden.

Die Tiefe der Ausnehmung beträgt vorzugsweise ungefähr die Hälfte des Durchmessers oder der Dicke eines Clip-Schenkels oder mehr.

Insbesondere beträgt die Tiefe der Ausnehmung in etwa die Dicke oder den Durchmesser eines Clip-Schenkels.

In Richtung der Längsrichtung der Schenkel der Clips dehnen sich die Ausnehmungen bei einem bevorzugten erfindungsgemäßen Clip-Applikator über eine Länge aus, die ungefähr dem Zweifachen des Durchmessers oder der Dicke eines Clip-Schenkels oder mehr entspricht.

Weiter bevorzugt beträgt die Länge der Ausnehmung etwa das Vierfache des Durchmessers oder der Dicke des Clip-Schenkels oder weniger. Dadurch wird sichergestellt, dass der Clip auch unter Belastung in seiner vorgesehenen Aufnahmeposition in dem Anlagewerkzeug bleibt und nicht unzulässig nach proximal ausweichen kann.

Die Gesamtlänge des Aufnahmebereichs für den Clip in den Werkzeugbacken entspricht vorzugsweise etwa der Länge der Clip-Schenkel vom Scheitelpunkt des V- oder U-förmigen Clips bis zum jeweiligen freien Ende entlang von deren Verlauf gemessen. Diese Länge ist größer als die Längsausdehnung des Clips und mit dieser nicht zu verwechseln.

Während der Aufnahmebereich proximal vorzugsweise einen Anschlag für den Verbindungsbereich (Scheitelpunkt) des Clips bildet, wird am distalen Ende des Aufnahmebereichs nicht in allen Fällen ein Anschlag für die freien Enden der Clip-Schenkel vorgesehen.

Ferner sind die Aufnahmebereiche vorzugsweise zumindest im Bereich der Ausnehmungen, die die Verbindungsbereiche aufnehmen, seitlich, d.h. in Querrichtung zur Clipebene, erweitert oder offen, so dass sich der Materialaufwurf, der in den Verbindungsbereichen beim Schließen der Clips entsteht, dort ohne Zwänge ausbilden kann.

Die Anlageflächen der Werkzeugbacken sind mit einem Führungselement versehen.

Das Führungselement umfasst einen oder mehrere längliche Vorsprünge. Diese länglichen Vorsprünge sind dann parallel zur Längsrichtung der Anlageflächen für die Clip-Schenkel angeordnet, so dass die Doppelsteg- und Mehrfachclips mit ihren mehreren Schenkeln parallel geführt und orientiert bleiben, wenn die Werkzeugbacken aus ihrer offenen Ruhestellung in die Schließstellung überführt werden.

Das Anlagewerkzeug kann zangenförmig mit gegeneinander verschwenkbar gelagerten Werkzeugbacken ausgebildet sein. Alternativ ist ein gabelförmig ausgebildetes Anlegewerkzeug möglich, bei dem die Werkzeugbacken an freien Enden elastisch verformbarer Gabelzinken angeordnet sind, wie dies beispielsweise in der DE 2845213 gezeigt ist.

Diese und weitere Vorteile der Erfindung werden im Folgenden anhand der Zeichnungen noch näher erläutert. Es zeigen im Einzelnen:
- Figur 1: einen offenbarten Clip-Applikator in Schnittdarstellung:
- Figur 2: ein Detail des Clip-Applikators der Figur 1;
- Figur 3: eine schematische Darstellung des Schließvorgangs bei einem Clip unter Verwendung eines offenbarten ClipApplikators;
- Figur 4A-C: ein Detail eines erfindungsgemäßen chirurgischen ClipApplikators beim Applikationsvorgang eines Clips;
- Figur 4D: ein mit einem erfindungsgemäßen Clip-Applikator geschlossenen Clip; und
- Figur 5A und 5B: alternative Ausführungsformen eines chirurgischen ClipApplikators im Detail.

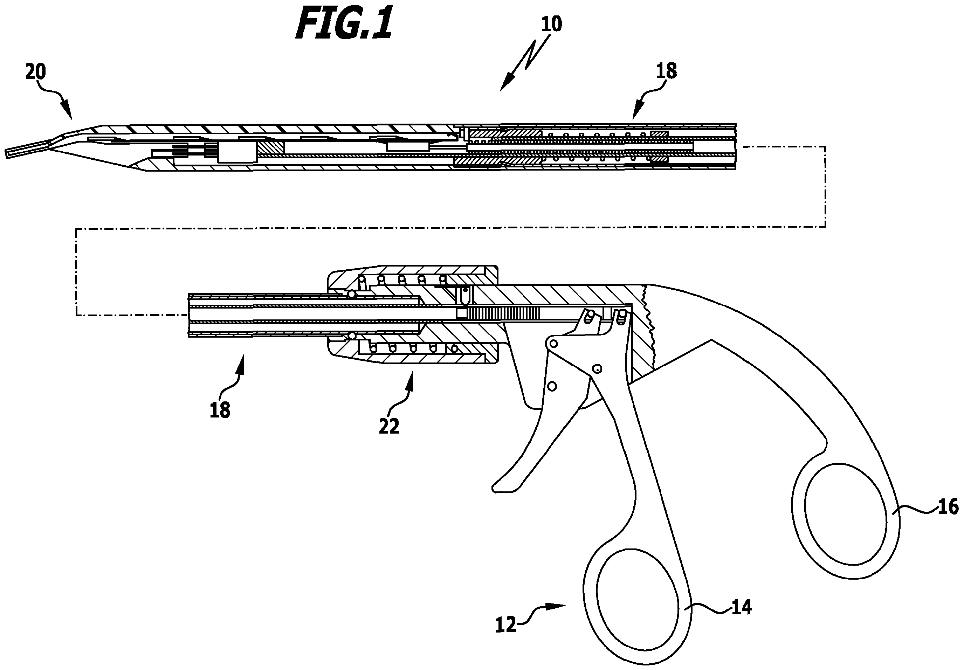

Figur 1 zeigt einen offenbarten chirurgischen Clip-Applikator, der in seinem grundlegenden Aufbau sich an dem Clip-Applikator orientiert, wie er in der DE 19603889 A1 im Zusammenhang mit der dort enthaltenen Figur 1 beschrieben ist.

Der Clip-Applikator 10 umfasst einen Griff 12 mit zwei Griffbranchen 14, 16, einen sich daran anschließenden Schaft 18 und ein am freien Ende des Schafts 18 angeordnetes Anlegewerkzeug 20. Der Griff 12 ist vorzugsweise drehbar und lösbar an dem Schaft 18 mittels eines Kupplungsstücks 22 gehalten.

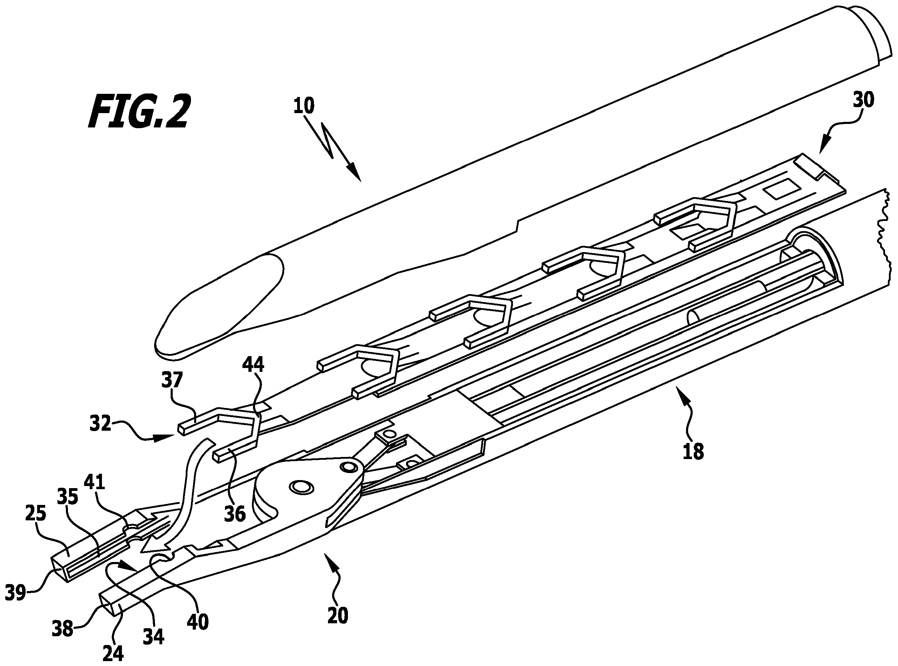

Bei der in den Figuren 1 und 2 gezeigten Ausführungsform des offenbarten chirurgischen Clip-Applikators 10 ist das am freien Ende des Schafts 18 angeordnete Anlegewerkzeug 20 zangenförmig ausgebildet und weist zwei gegeneinander schwenkbar gelagerte Werkzeugbacken 24, 25 auf. Der Schaft 18, der hier als Rohrschaft ausgebildet ist, kann an seinem dem freien Ende benachbarten Abschnitt ein Clip-Magazin 30 aufnehmen, das die zu applizierenden Clips dem Anlegewerkzeug 20 vereinzelt zuführbar vorrätig hält.

Über eine Betätigung des Griffs 12 kann ein Clip 32 aus dem Clip-Magazin 30 zum Anlegewerkzeug vorgeschoben werden, wo er in Aufnahmebereichen 34, 35 der Werkzeugbacken 24, 25 geführt aufgenommen und zur Applikation an einem Gewebeabschnitt, beispielsweise ein Blutgefäß, bereitgehalten wird.

Die Aufnahmebereiche 34, 35 sind als Nuten ausgebildet, in die der Clip 32 mit seinen Schenkeln 36, 37 gleitend eingeführt werden kann. Bzgl. der genaueren Funktion des Vorschubmechanismus darf wiederum auf die Beschreibung der Figuren 1 bis 3 der DE 19603889 voll umfänglich Bezug genommen werden.

Der Aufnahmebereich 34, 35 der Werkzeugbacken 24, 25 ist an seinem distalen Ende mittels einer Wand 38, 39 geschlossen, während er an seinem davon entfernt liegenden Ende jeweils mit einer Ausnehmung 40, 41 endet, die beim Schließen der Werkzeugbacken 24, 25 einen Verbindungsbereich 44 des Clips 32 aufnehmen, der die beiden Schenkel 36, 37 des V-förmigen Clips 32 miteinander verbindet.

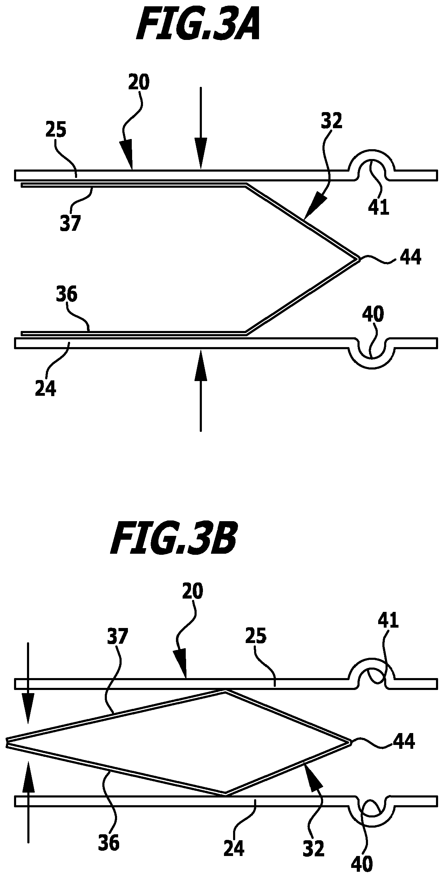

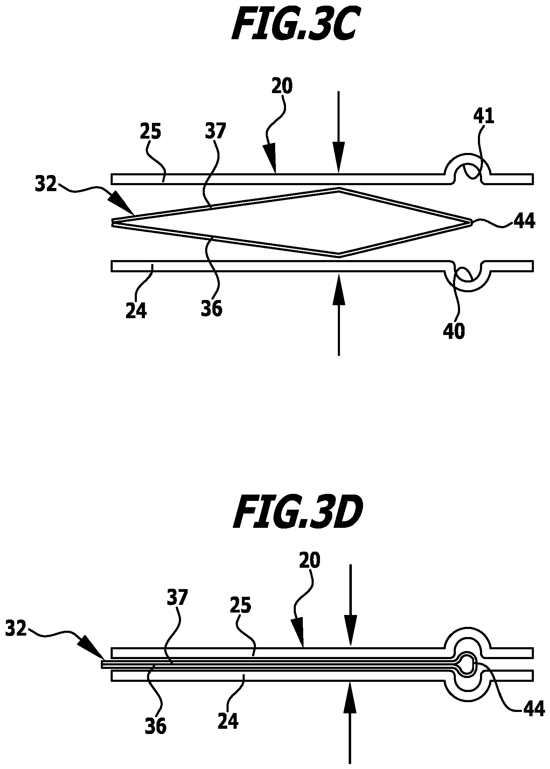

Der Schließvorgang des Clips mit einer Überführung der Werkzeugbacken 24, 25 von der offenen Ruhestellung in die Schließstellung sei anhand der schematischen Figur 3 beschrieben.

Figur 3A zeigt nochmals den Clip 32 im Werkzeug 20 von den Werkzeugbacken 24, 25 aufgenommen. Danach werden durch eine Schließbewegung, die durch die beiden Pfeile in Figur 3A angedeutet ist, die beiden Werkzeugbacken 24, 25 aus der offenen Ruhestellung der Figur 3A in eine Schließstellung der Figur 3D überführt.

Die beiden Schenkel 36, 37 werden dabei einander angenähert, wobei sie zunächst um den Verbindungsbereich oder Scheitelpunkt 44 verschwenkt werden. Diese Situation ist in der Figur 3B gezeigt, bei der durch die Schwenkbewegung der Schenkel 36, 37 deren freie Enden aneinander zur Anlage kommen.

Bei der Fortsetzung des Schließvorgangs wie er in den Figuren 3C und 3D veranschaulicht ist, werden nun die beiden Schenkel 36, 37 verformt und an dem Verbindungsbereich 44 tritt ein deutlicher Materialaufwurf auf, der einen erhöhten Kraftaufwand bei der weiteren Schließung des Clips erfordert.

Aufgrund der offenbarten Ausgestaltung der Aufnahmebereiche der Werkzeugbacken 24, 25 kann der Clip 32 vollständig geschlossen werden, ohne dass die dem Verbindungsbereich 44 direkt benachbarten Abschnitte der Schenkel 36, 37 direkt aneinander angelegt werden müssen. Vielmehr lassen die Ausnehmungen 40, 41 der Werkzeugbacken 24, 25 im Anschluss an die Aufnahmebereichen 34, 35 zu, dass benachbart zum Verbindungsbereich die Clip-Schenkel etwas auf Abstand voneinander gehalten bleiben und nicht vollständig verpresst werden. Das im Verbindungsbereich erzielte Spaltmaß ist damit größer als das zwischen den Schenkeln geforderte Spaltmaß.

Dadurch wird ein geringerer Materialaufwurf erzeugt und die Schenkel 36, 37 des Clips lassen sich einfacher in ihre endgültige Schließstellung und das dort vorgegebene Spaltmaß von beispielsweise ca. 0,25 mm bringen.

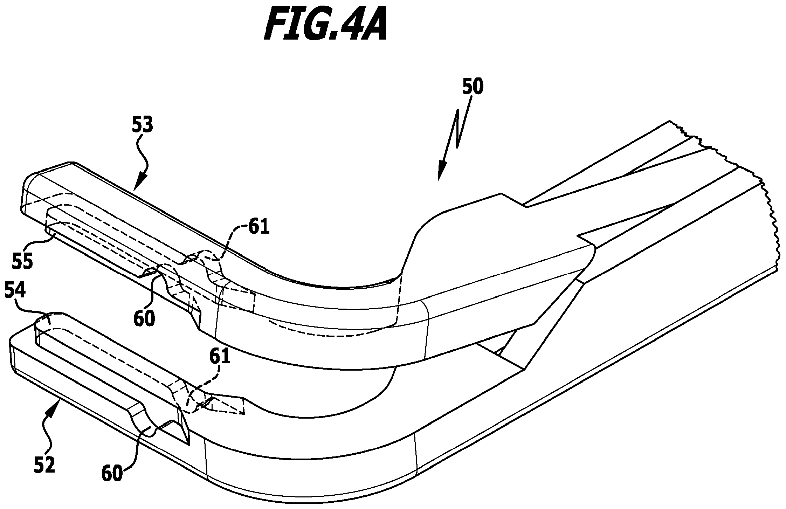

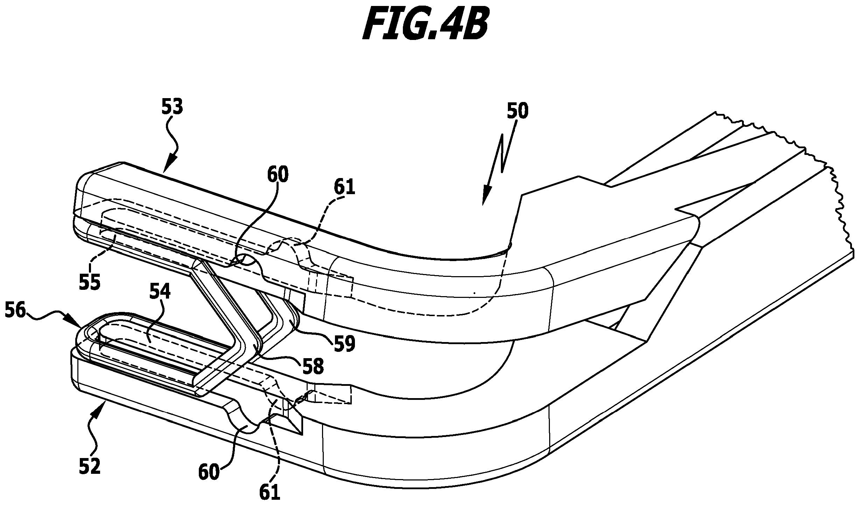

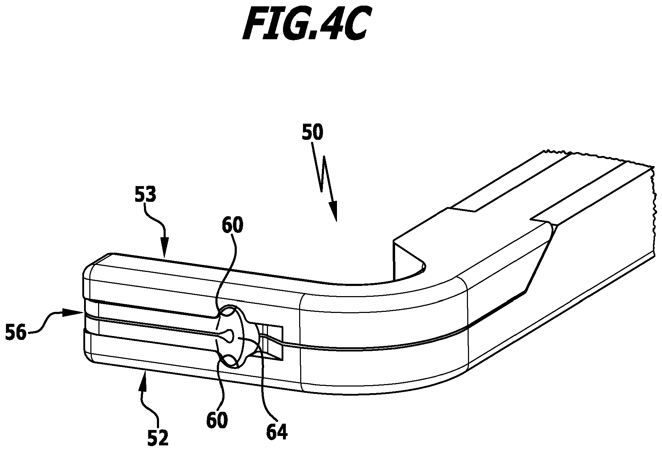

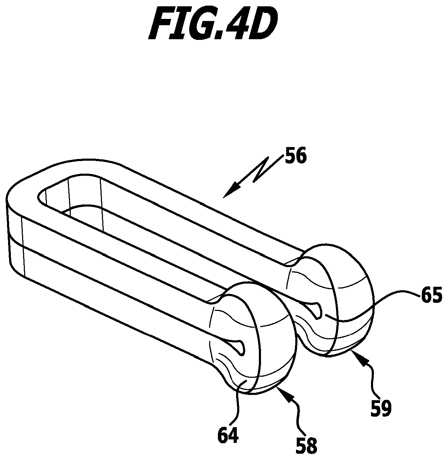

Der in den Figuren 3A bis 3D schematisch gezeigte Vorgang ist anhand der Figuren 4A bis 4D nochmals anhand einer erfindungsgemäßen Ausführungsform eines chirurgischen Clip-Applikators bzw. dessen Anlegewerkzeug 50 im Einzelnen und im Zusammenhang mit dem Applizieren eines Doppelstegclips 56 gezeigt. Das Anlegewerkzeug 50 der Figur 4 unterscheidet sich von dem Anlegewerkzeug 20 der Figuren 1 und 2 dadurch, dass die Werkzeugbacken gegenüber dem (nicht dargestellten) Griff in einem Winkel von ungefähr 90° abgewinkelt sind und dass erfindungsgemäß die Aufnahmebereiche der Werkzeugbacken 52, 53 anstelle einer Nut mit in Längsrichtung der Werkzeugbacken 52, 53 verlaufenden Vorsprüngen 54, 55 versehen sind, die der Führung eines sogenannten Doppelstegclip 56, bei dem die freien Clipenden miteinander verbunden sind, dienen. Von dem freien Ende der Werkzeugbacken 52, 53 zurückgesetzt sind in den Abschnitten, in denen ein Verbindungsbereich 58, 59 des Doppelstegclips 56 beim Schließen des Clips 56 zur Anlage kommt, Ausnehmungen 60, 61 vorgesehen, die vermeiden, dass die Verbindungsbereiche 58, 59 beim Schließen des Doppelstegclips 56 auf dasselbe Spaltmaß verpresst werden müssen wie die Clip-Schenkel.

Die Situation des vollständig geschlossenen Clips 56 im Anlegewerkzeug 50 ist in der Figur 4C dargestellt. In vergrößerter Darstellung enthält Figur 4D noch mal den geschlossenen Doppelstegclip 56 bei dem im Verbindungsbereich 58, 59 ein Aufwurf 64, 65 zu erkennen ist.

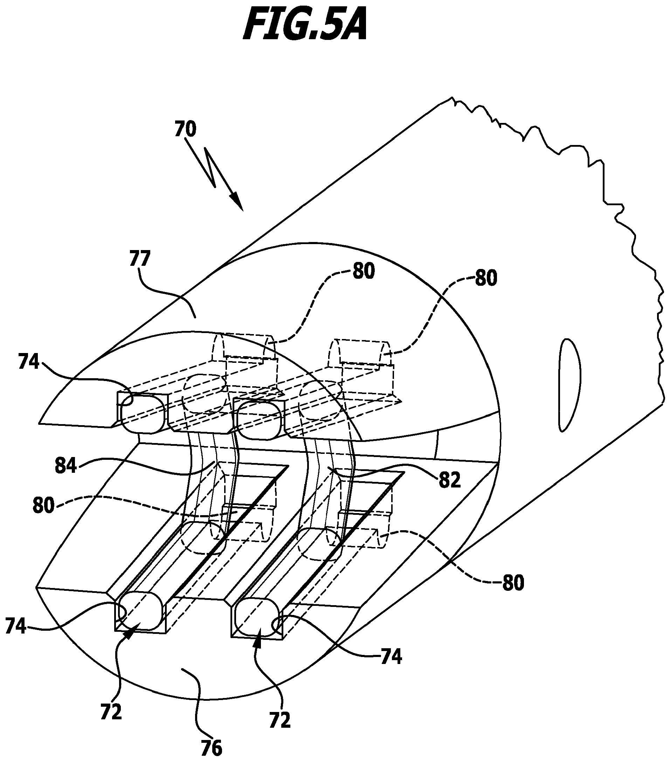

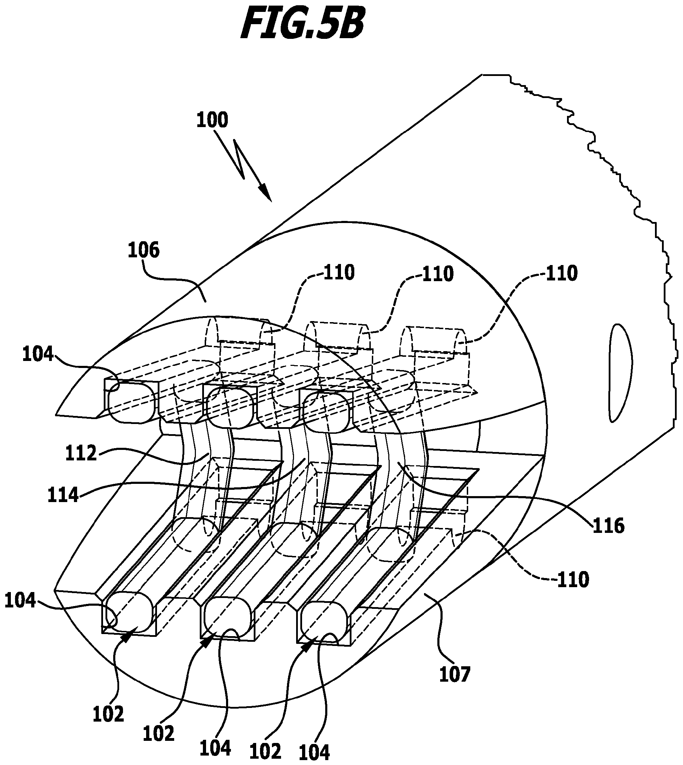

In den Figuren 5A und 5B sind zwei weitere alternative Ausführungsformen des erfindungsgemäßen chirurgischen Clip-Applikators mit Anlegewerkzeugen 70 bzw. 100 gezeigt, bei denen ein Doppelstegclip 72 bzw. ein Dreifachclip 102 in zwei bzw. drei Nuten 74, 104 aufgenommen ist.

Die Nuten 74; 104 der Werkzeugbacken 76, 77; 106, 107 der Anlegewerkzeuge 70 bzw. 100 fungieren als Aufnahmebereiche, die sich in Ausnehmungen 80; 110 aufweiten, so dass die Verbindungsbereiche 82, 84; 112, 114, 116 der freien Schenkel der Doppelsteg- bzw. Dreifachclips 72; 102 beim Schließvorgang dort aufgenommen werden und ausweichen können, so dass die Verbindungsbereiche nicht auf ein für die Clip-Schenkel vorgegebenes Spaltmaß verpresst werden müssen.

Im Fall der Führung der Clips in Nuten als Aufnahmebereiche ist es generell vorteilhaft, wenn sich die Ausnehmungen, die die Verbindungsbereiche der freien Schenkel der Clips aufnehmen, auch in Querrichtung erweitern, so dass die Bildung des Aufwurfs seitlich der einzelnen Clips (vgl. Figur 4D) ohne Zwang erfolgen kann. Diese führt zu einen weiteren Erleichterung des Schließvorgangs der Clips.

## Patentansprüche

1. Chirurgischer Clip-Applikator (10) mit einem Doppelsteg- oder Mehrfachclip mit einer U- oder V-förmigen Clipstruktur mit jeweils zwei über einen Verbindungsbereich miteinander verbundenen Schenkeln,
wobei der Applikator (10) einen Griff (12), einen sich daran anschließenden Schaft (18) und ein Anlegewerkzeug (50; 70; 100) am freien Ende des Schafts (18) umfasst, welches mit dem Griff (12) betätigbar ist und zwei beim Betätigen aus einer offenen Ruhestellung in eine Schließstellung überführbare Werkzeugbacken (52, 53; 76, 77; 106, 107) aufweist, die jeweils einen Aufnahmebereich für den zu applizierenden Clip definieren, wobei die Aufnahmebereiche für den zu applizierenden Clip jeweils eine Anlagefläche für die jeweiligen Schenkel, welche sich zumindest über einen Teil der Länge der Schenkel erstreckt, und Ausnehmungen (60, 61; 80; 110) aufweisen, in denen die Verbindungsbereiche der Schenkel des Clips in der Schließstellung der Werkzeugbacken (52, 53; 76, 77; 106, 107) angeordnet sind, wobei die Anlageflächen ein Führungselement umfassen, **dadurch gekennzeichnet, dass** das Führungselement mindestens einen länglichen Vorsprung (54, 55) umfasst, welcher parallel zur Längsrichtung der Anlageflächen für die Clip-Schenkel und zwischen den Clip-Schenkeln angeordnet ist, so dass die Doppelsteg-
und Mehrfachclips mit ihren mehreren Schenkeln parallel geführt und orientiert bleiben, wenn die Werkzeugbacken aus ihrer offenen Ruhestellung in die Schließstellung überführt werden.

2. Clip-Applikator nach Anspruch 1, **dadurch gekennzeichnet, dass** die Anlageflächen im Bereich der freien Enden der Schenkel einen Rücksprung aufweisen.

3. Clip-Applikator nach Anspruch 2, **dadurch gekennzeichnet, dass** die Anlageflächen so bemessen sind, dass die Schenkel in der Schließstellung der Werkzeugbacken (52, 53; 76, 77; 106, 107) nur mit einem mittleren Abschnitt an den Werkzeugbacken (52, 53; 76, 77; 106, 107) anliegen.

4. Clip-Applikator nach Anspruch 3, **dadurch gekennzeichnet, dass** die Anlageflächen so bemessen sind, dass die Schenkel über ca. ein Drittel ihrer Länge oder mehr, insbesondere die Hälfte ihrer Länge oder mehr in der Schließstellung der Werkzeugbacken (52, 53; 76, 77; 106, 107) anliegen.

5. Clip-Applikator nach Anspruch 4, **dadurch gekennzeichnet, dass** die Anlageflächen so bemessen sind, dass die Schenkel über ca. zwei Drittel ihrer Länge oder mehr in der Schließstellung der Werkzeugbacken (52, 53; 76, 77; 106, 107) anliegen.

6. Clip-Applikator nach einem der Ansprüche 1 bis 5, **dadurch gekennzeichnet, dass** die Ausnehmung senkrecht zur Schließrichtung des Clip-Schenkels eine Tiefe aufweist, die ca. dem Durchmesser oder der Dicke eines Clip-Schenkels oder mehr entspricht.

7. Clip-Applikator nach Anspruch 6, **dadurch gekennzeichnet, dass** die Länge der Ausnehmung parallel zur Längsrichtung des Clip-Schenkels etwa dem Zweifachen des Durchmessers oder der Dicke eines Clip-Schenkels oder mehr entspricht.

8. Clip-Applikator nach Anspruch 7, **dadurch gekennzeichnet, dass** die Länge der Ausnehmung etwa dem Vierfachen des Durchmessers oder der Dicke eines Clip-Schenkels oder weniger entspricht.

## Claims

1. Surgical clip applicator (10) with a double-shank or multiple clip having a U-shaped or V-shaped clip structure having each two legs connected to each other by a connecting region, wherein the applicator (10) comprises a handle (12), an adjoining shaft (18) and at the free end of the shaft (18) an applying tool (50; 70; 100), which is actuatable with the handle (12) and has two tool jaws (52, 53; 76, 77; 106, 107) which are transferable from an open, idle position to a closed position when actuated and each define a receiving area for the clip to be applied, wherein the receiving areas for the clip to be applied each have an abutment surface for the respective legs, which extends at least over part of the length of the legs, and recesses (60, 61; 80; 110) in which the connecting regions of the legs of the clip are arranged when the tool jaws (52, 53; 76, 77; 106, 107) are in the closed position, wherein the abutment surfaces comprise a guide element, **characterized in that** the guide element has at least one elongate projection (54, 55), which is arranged parallel to the longitudinal direction of the abutment surfaces for the clip legs and between the clip legs, so that the double-shank and multiple clips with their plurality of legs remain guided and oriented in parallel when the tool jaws are transferred from their open, idle position to the closed position.

2. Clip applicator in accordance with claim 1, **characterized in that** the abutment surfaces have a setback in the area of the free ends of the legs.

3. Clip applicator in accordance with claim 2, **characterized in that** the abutment surfaces are of such dimensions that the legs abut only with a middle section against the tool jaws (52, 53; 76, 77; 106, 107) when the tool jaws (52, 53; 76, 77; 106, 107) are in the closed position.

4. Clip applicator in accordance with claim 3, **characterized in that** the abutment surfaces are of such dimensions that the legs abut over approximately a third of their length or more, in particular, half of their length or more, when the tool jaws (52, 53; 76, 77; 106, 107) are in the closed position.

5. Clip applicator in accordance with claim 4, **characterized in that** the abutment surfaces are of such dimensions that the legs abut over approximately two thirds of their length or more when the tool jaws (52, 53; 76, 77; 106, 107) are in the closed position.

6. Clip applicator in accordance with any one of claims 1 to 5, **characterized in that** the recess has a depth perpendicular to the closing direction of the clip leg, which corresponds approximately to the diameter or thickness of a clip leg or more.

7. Clip applicator in accordance with claim 6, **characterized in that** the length of the recess parallel to the longitudinal direction of the clip leg corresponds approximately to twice the diameter or thickness of a clip leg or more.

8. Clip applicator in accordance with claim 7, **characterized in that** the length of the recess corresponds approximately to four times the diameter or thickness of a clip leg or less.

## Revendications

1. Applicateur de clip chirurgical (10) avec un clip à double traverse ou multiple avec une structure de clip en forme de U ou de V chacun avec deux branches reliées l'une à l'autre via un domaine de liaison, où l'applicateur (10) comprend une poignée (12), une tige (18) faisant suite à celle-ci et un outil de mise en place (50; 70; 100) à l'extrémité libre de la tige (18), qui peut être actionné avec la poignée (12) et présente deux mâchoires d'outil (52, 53; 76, 77; 106, 107) qui peuvent être transférées d'une position de repos ouverte à une position fermée lors de l'actionnement, qui définissent chacune un domaine de réception pour le clip à appliquer, où les domaines de réception pour le clip à appliquer présentent chacun une surface de contact pour les branches respectives, qui s'étend au moins sur une partie de la longueur des branches, et des évidements (60, 61; 80; 110) dans lesquels les domaines de liaison des branches du clip sont disposés dans la position fermée des mâchoires d'outil (52, 53; 76, 77; 106, 107), où les surfaces de contact comprennent un élément de guidage, **caractérisé en ce que** l'élément de guidage comprend au moins une saillie allongée (54, 55) qui est disposée parallèlement à la direction longitudinale des surfaces de contact pour les branches de clip et entre les branches de clip de sorte que les clips à double traverse et multiples restent guidés et orientés parallèlement avec leurs multiples branches lorsque les mâchoires d'outil sont transférées de leur position de repos ouverte à la position fermée.

2. Applicateur de clip selon la revendication 1, **caractérisé en ce que** les surfaces de contact présentent un retrait dans le domaine des extrémités libres des branches.

3. Applicateur de clip selon la revendication 2, **caractérisé en ce que** les surfaces de contact sont dimensionnées de telle sorte que les branches dans la position fermée des mâchoires d'outil (52, 53; 76, 77; 106, 107) ne sont en contact avec les mâchoires d'outil (52, 53; 76, 77; 106, 107) que par une section moyenne.

4. Applicateur de clip selon la revendication 3, **caractérisé en ce que** les surfaces de contact sont dimensionnées de telle sorte que les branches sont en contact sur environ un tiers de leur longueur ou plus, notamment la moitié de leur longueur ou plus, dans la position fermée des mâchoires d'outil (52, 53; 76, 77; 106, 107).

5. Applicateur de clip selon la revendication 4, **caractérisé en ce que** les surfaces de contact sont dimensionnées de telle sorte que les branches sont en contact sur environ deux tiers de leur longueur ou plus dans la position fermée des mâchoires d'outil (52, 53; 76, 77; 106, 107).

6. Applicateur de clip selon l'une des revendications 1 à 5, **caractérisé en ce que** l'évidement présente perpendiculairement à la direction de fermeture de la branche de clip une profondeur qui correspond approximativement au diamètre ou à l'épaisseur d'une branche de clip ou plus.

7. Applicateur de clip selon la revendication 6, **caractérisé en ce que** la longueur de l'évidement parallèlement à la direction longitudinale de la branche de clip correspond approximativement au double du diamètre ou de l'épaisseur d'une branche de clip ou plus.

8. Applicateur de clip selon la revendication 7, **caractérisé en ce que** la longueur de l'évidement correspond approximativement à quatre fois le diamètre ou l'épaisseur d'une branche de clip ou moins.
